# EUROPEAN PATENT APPLICATION

(11) **EP 4 163 370 A1**
(43) Date of publication of application: **12.04.2023**
(21) Application number: 21201709.9
(22) Date of filing: 08.10.2021
(51) Int. Cl.: C12N 15/10, C12Q 1/6806

(54) **STABILIZATION OF NUCLEIC ACIDS IN BIOLOGICAL SAMPLES**

(71) Applicant: AXAGARIUS GmbH & Co. KG, 52355 Düren (DE)
(72) Inventor: Wyrich, Ralf, 41516 Grevenbroich (DE)
(74) Representative: dompatent von Kreisler Selting Werner - Partnerschaft von Patent- und Rechtsanwälten mbB

(57) **Abstract**

The present invention relates to the stabilization of nucleic acid-containing crude sample materials for allowing its separation by separation methods. Stabilizing solutions containing mixtures of chaotropic salts suitable for said purpose are provided herewith. Furthermore, provided are nucleic acid separation kits and sample collection containers comprising such stabilizing solutions.

## Description

The present invention relates to the stabilization of nucleic acid-containing crude sample materials for allowing its separation by separation methods. Stabilizing solutions containing mixtures of chaotropic salts suitable for said purpose are provided herewith. Furthermore, provided are nucleic acid separation kits and sample collection containers comprising such stabilizing solutions.

### Background of the Invention

The present invention covers the field of preanalytical sample handling, sample collection and transport, for molecular biology applications. In order to keep the integrity of molecular analytes like DNA and RNA intact, the use of stabilization solutions is necessary to prevent degradation and as a consequence false negative results in a subsequent analytical assay. As the collection of a biological sample often takes place in poorly controlled environments, it is desirable that stabilization solutions used for sample collection are nonhazardous. From a production standpoint it is moreover desirable, that the stabilization solution is composed of only few components which are easy to proof with routine quality control measurements like pH, density or conductivity.

The stabilization of nucleic acids, such as DNA and RNA, in biological samples is crucial for state-of-the-art diagnostic applications like PCR, RT-PCR or qPCR. The current diagnostic procedures rely on the detection of genetic material, e.g. the detection of mutations in the genotype for the investigation of a cancer disease or a genetic disposition, or the identification of a pathogen in case of an infectious disease. In any case, the reliable result of a diagnostic test relies on the quality of the sample material that was used for the analysis. As the biological samples, used for diagnostic procedures are normally taken at remote places, e.g. at the doctor's office and need to be shipped to the laboratory that performs the analysis, the sample collection and transport needs to be performed under conditions, which prevent that the genetic material undergoes changes. Nucleic acids can degrade during transport for example, if the sample material is not stabilized properly. In a diagnostic procedure, targeting e.g. a specific pathogen, this can lead to a false negative result, if the DNA or RNA of the pathogen is degraded during transport. It is therefore desirable to have a transport medium that stabilizes nucleic acid material after a sample has been collected until the nucleic acid is extracted in the analysis laboratory. Therefore, there is a need for a stabilization solution that stabilizes RNA and DNA during the preanalytical phase (collection and transport of a sample) until the analytical phase (extraction of the nucleic acid and detection) of the diagnostic workflow. In the specific field of the veterinary diagnostic, it is especially desirable that a stabilization solution is non-hazardous, as a veterinary who needs to take a specimen from a livestock in a stable or at a meadow does not have a controlled environment, where hazardous reagents can be handled under the due safety regulations. Therefore, there is a specific need for a stabilization solution, which stabilizes DNA and RNA in a biological sample but does not require special safety precautions during handling.

The state-of-the-art technologies applying chaotrophic agents to stabilize nucleic acids in biological samples use complex mixtures, consisting of several components. The manufacturing of such complex mixtures in routine production processes is error prone. Routine quality control procedures for liquid mixtures rely on pH, density and conductivity measurement. US020110027862 describes a mixture of "no more than 20 mM" of a metal ion in a background of 2-8 M guanidinium salt. A mistake during manufacturing will not be detectable with routine procedures, but only with quantitative measure of the individual compound. Such quantitative measures are costly, time consuming, and hamper lean production processes. The same applies to complex mixtures described in EP2206792, US020050227225, EP3020832 or EP3205722. A false net weight of a single component in a complex mixture of components is difficult to detect by ph, density or conductivity.

Moreover, most of the state-of-the-art stabilizing compositions use a single guanidinum salt but the concentration of this salt is very high. The common guanidinium salts, guanidinium hydrochloride and guanidinium thiocyanate are hazardous components that need to be declared above a certain concentration and safety measures during handling must be taken accordingly. In contrast to a well-controlled environment like a laboratory, handling of such reagents in poorly controlled environments like e.g. a farm, where specimen from livestock are taken in a stable or at a meadow can be critical.

Many state-of-the-art stabilization solutions rely on chaotrophic agents like guanidinium salts in combination with other components in order to stabilize nucleic acids in biological material:
PreAnalytiX (EP 2206792) describes a guanidinium salt, a buffering agent and a detergent.

Roche Molecular Systems (US 2005/0227225) describes the use of a combination of a chaotrophic agent, an organic solvent, a reducing agent, a buffering agent and a detergent.

Longhorn Vaccines & Diagnostics (EP 3020832) describes a mixture of a) one or more chaotropes; b) one or more detergents; c) one or more reducing agents; d) one or more chelators; and e) one or more surfactants.

Sarstedt (EP 3205722) describes an aqueous solution consisting of a. at least one guanidinium salt, b. at least one buffering agent for buffering to a pH of 5.0 to 8.0, c. at least one nonionic detergent and d. at least one complexing agent for divalent cations, e. and optionally proteinase and/or DNase, and f. is free from reduction agents.

Life Technologies (US 2011/0027862) describes a combination of guanidine and a metal ion from a group 1 or group 2 metal.

All applications describe the use of complex mixtures, containing chaotropic agents and other components to stabilize nucleic acids in biological samples.

A stabilization solution for the preservation of nucleic acids in biological samples that is non-complex in composition and not hazardous in handling was needed for sample collection in poorly controlled environments.

### Short Description of the Invention

There was a need for a stabilization solution, which was less complex in composition to ensure a reliable, less error prone production and which was nonhazardous in order to simplify the handling and expand the applicability to less controlled environments.

It was now found that a solution with concentrations below 2,4 M for each of the two guanidinium salts, guanidinium hydrochloride and guanidinium thiocyanate that are considered as nonhazardous, provides for the desired stabilization of biological samples. Such a solution that contains 2,4 M guanidinium thiocyanate or less and 2,4 M guanidinium hydrochloride or less does not need to be handled with special precautions. It was now found that even the use of lower amounts of the two different guanidine salts are suitable to stabilize nucleic acids in biological samples. Such stabilization compositions can be used to collect biological sample material in poorly controlled environments like farms for livestock production.

The invention thus provides:
(1) A method for stabilizing and isolating nucleic acids from a nucleic acid-containing biological sample which comprises adding to said sample, prior to subjecting it to a separation procedure, a sufficient amount of a stabilizing solution comprising a mixture of at least two chaotropic salts, preferably the sum of the molarity of said at least two chaotropic salts in the stabilizing solution is at least 2.0 M, most preferably at least 2.2 M.
(2) In a preferred embodiment of aspect (1), the method comprises
   (a) providing the nucleic acid-containing sample,
   (b) stabilizing the sample of step (a) by adding a sufficient amount of the stabilizing solution, optionally prior to, concomitant with, and/or after lysing the sample by the addition of a distinct lysis buffer and/or lysis solution;
   (c) loading the stabilized sample of step (b) to a separation material to bind the nucleic acids to the separation material;
   (d) washing the separation material with bound nucleic acids by adding one or more wash buffers to remove unwanted materials; and
   (e) releasing the nucleic acids from the separation material by applying a release solution.
(3) In a preferred embodiment of aspect (1) the separation material is magnetic beads, step (d) comprises separating the beads from the sample by magnetic bead separation; and washing the separated beads with one or more wash buffers, and step (e) comprises releasing the nucleic acids from the separated beads by applying a release solution
(4) A stabilizing solution as defined in aspects (1) to (3).
(5) A sample collection container comprising or being coated with the stabilizing solution as defined in aspects (1) to (3).
(6) A kit comprising the stabilizing solution of aspect (4) and/or the sample collection container of aspect (5).
(7) The use of the stabilizing solution of aspect (4) or the sample collection container of aspect (5) for stabilizing nucleic acids in biological samples.
(8) The use of a kit of aspect (6) for stabilizing nucleic acids in or isolating nucleic acids from biological samples.

The present invention overcomes the state-of-the-art disadvantages of complex composed solutions and the use of hazardous components at concentrations that make safety precautions during handling necessary. The mixture of only two guanidinium salts, both at concentrations below the critical value for hazardous components, are utilized in the methods, stabilization solutions and kits of the present invention. Advantages compared to the state of the art are therefore less error prone production processes and handling without the need for special safety precautions.

### Detailed Description of the Invention

The method for stabilizing and isolating nucleic acids from a nucleic acid-containing biological sample according to aspects (1) to (3) comprises the addition a sufficient amount of a stabilizing solution comprising a mixture of at least two chaotropic salts (hereinafter also referred to as "stabilizing solution of the invention") to said sample.

"Biological samples" according to the invention include material directly obtained from biological material (such as cells, tissue and bodily fluids) and also synthetic or semi-synthetic material.

"Nucleic acids" according to the present invention includes DNA and RNA and derivatives thereof.

"Chaotropic salts" according to the invention are salts according to the Hofmeister series including, but not limited to barium, calcium, sodium and guanidinium perchlorates, thiocyanates, isothiocyanates, iodides and perchloracetates, and guanidinium hydrochlorid. A particularly preferred stabilizing solution preferably contains a mixture of guanidinium thiocyanate and guanidinium hydrochloride.

Further, a stabilizing solution of the invention contains the alt least two chaotropic salts, at concentrations, selected independently from each other, from about 100 mM to about 5 M, preferably from about 500 mM to about 3 M, and most preferably from about 1.2 M to about 2.4 M.

Alternatively, the stabilizing solution of the invention may contain at least three chaotropic salts from those defined above, preferably contains a mixture of guanidinium thiocyanate, guanidinium hydrochloride and sodium perchlorate, with concentrations as defined above.

It is preferred that the sum of the molarity of said at least two chaotropic salts in the lysis solution is at least 2.2 M, preferably at least 2.4 M.

Adding the stabilizing solution to the sample in a sufficient amount according to the invention refers to the addition of a stabilizing solution in an amount equal or greater than the volume of the sample, preferably about three-times the volume of the sample. The stabilizing solution may further comprise buffers, salts, chelators, detergents, and/or reducing agents.

In a particular embodiment the stabilizing solution comprises guanidinium thiocyanate (GTC) and guanidinium hydrochloride (GuHCl), independently from each other at concentrations from about 1.2 M to about 2.4 M. The following equimolar formulations of guanidinium thiocyanate and guanidinium hydrochloride were tested against the single salts:
2.4 M GTC and 2.4 M GuHCl (such equimolar formulations being in the following shortly referred to as 2.4 M GTC/GuHCl), 2.0 M GTC/GuHCl, 1.6 M GTC/GuHCl, 1.2 M GTC/GuHCl, 1.0 M GTC/GuHCI and 0.8 M GTC/GuHCI (all formulations buffered with 200 mM sodium acetate pH 6.0), among which 2.4 M GTC/GuHCl, 2.0 M GTC/GuHCl, 1.6 M GTC/GuHCI and 1.2 M GTC/GuHCl, were found as providing for an improvement over the reference.

In a further particular embodiment, the stabilizing solution contains guanidinium thiocyanate, guanidinium hydrochloride and sodium perchlorate (SPC), independently from each other at concentrations from about 0.8 M to about 2.4 M. The following formulations with guanidinium thiocyanate, guanidinium hydrochloride and sodium perchlorate were tested: 0.8 M GTC/GuHCl/SPC, 0.8 M GTC/GuHCl/ 1.2 M SPC, 0.8 M GTC/GuHCl/ 1.6 M SPC and 0.8 M GTC/GuHCl/ 2.0 M SPC (all formulations buffered with 200 mM sodium acetate pH 6,0), among which 0.8 M GTC/GuHCl/ 1.2 M SPC, 0.8 M GTC/GuHCl/ 1.6 M SPC and 0.8 M GTC/GuHCl/ 2.0 M SPC were found as providing for an improvement over the reference.

The method of aspects (2) and (3) may, apart from stabilizing the sample in step (b) by adding a sufficient amount of the stabilizing solution, further comprise, prior to, concomitant with, and/or after said addition, the lysis of the sample material, e.g. by mechanical lysis, or by the addition of a distinct lysis buffer and/or lysis solution. Such lysis solution may comprise a lysing enzyme like a protease, the lysis buffer may (further) comprise ionic components, such as salts, chaotropic salts different from those of the stabilizing solution and ionic tensides like sodium dodecylsulfate (SDS) and non-ionic detergents such as EDTA.

The method of aspects (2) and (3) comprise in step (c) the loading of the stabilized sample to a separation material to therewith bind the nucleic acids to the separation material. "Separation materials" according to the invention include silica-based materials of various shapes and sizes including beads, membranes and surfaces. In the particular embodiment of aspect (3), the separation material is silica-based magnetic beads or carboxylated magnetic beads. Prior to or during said loading in step (c), the stabilized sample may further be subjected to a protease digest, e.g. with proteinase K, by adding a protease-containing solution and/or by adding a suitable binding solution. Such binding solution is generally a high salt (sodium perchlorate), buffered (Tris and and/or citrate buffer), surfactant-containing (Tween^{®} and/or sodium dodecylsulfate (SDS)) and ethanol-containing aqueous solution.

The method of aspects (2) and (3) may further include prior to loading the stabilized sample to the separation material or beads of step (c) the addition of an inhibitor removal solution comprising (1) a polyvinylpyrrolidone (PVP) component containing PVP, a derivative thereof, and/or monomers thereof, and (2) ammonium sulfate (AS). Such addition of the inhibitor removal solution may already occur prior to, concomitant with, or after the addition of the addition of the stabilizing solution of step (b). The inhibitor removal solution may contain the PVP component in an amount of about 2 to about 60 %(w/v), preferably in an amount of about 5 to about 30 %(w/v) and most preferably in an amount of about 8 to about 20 %(w/v), and/or the AS in an amount of about 50 mM to about 5 M, preferably in an amount of about 100 mM to about 1.5 M, and most preferably in an amount of about 500 mM to about 1 M. It is preferred that in the inhibitor removal solution the weight ratio of PVP component to the AS is from about 10:1 to 1:10, preferably from 5:1 to about 1:5, and most preferably from about 2:1 to about 1:3. The inhibitor removal solution may further comprise buffers, salts, chelators, detergents, reducing agents and/or stabilizing agents. It is preferred that the volume of the inhibitor removal solution added to the sample prior to step (c) is equal or greater than the volume of the sample or the stabilized sample for separation, preferably about three-times the volume of the sample or the stabilized sample for separation.

The PVP component comprises PVP with an average molecular weight of from about 1000 to about 500000 g/mol, preferably from about 2000 to about 50000 g/mol; and/or alkylated or halogenated derivatives of polyvinylpyrrolidone.

In the method of aspects (2) and (3), the wash buffers of step (d) are salt-containing and/or ethanol-containing aqueous solutions (e.g. 0-3 M sodium perchlorate , 80% ethanol 20% water, with 0-10 % (wt/vol) sodium acetate, 0-2 % (wt/vol)Tween^{®} 0-3 % (wt/vol) sorbitol and 0-9 % (wt/vol) acetic acid). The volume of each wash buffer applied shall be in the range of 10- to 50 fold of the volume of the separation material.

In the method of aspects (2) and (3), the release solution is water or an alkaline low-salt buffer (e.g. an aqueous solution containing 5 mM Tris/HCI). The volume of the release solution applied is the 1- to 10-fold of the separation material. Preferred embodiments of the stabilizing solution of aspect (4) and the sample collection container comprising or being coated with the stabilizing solution of aspect (5) are those defined for aspects (1) to (3) above. Such collection container is obtainable by filling a suitable amount of stabilizing solution into the container, optionally followed by a drying or freeze-drying step.

The kit comprising the stabilizing solution and/or the sample collection container of aspect (6) may further comprise one or more of the following components: lysis solutions/buffers, inhibitor removal solutions, washing solutions and release solutions, protease-containing solutions, binding solutions, and separation material/beads, including those specified above,.

The invention will be further explained by the following examples, which are not to be construed as limiting the invention.

### Examples

### Abbreviations:

- DNA: desoxyribonucleic acid
- RNA: ribonucleic acid
- PCR: polymerase chain reaction
- RT-PCR: reverse transcriptase PCR
- qPCR: quantitative PCR
- GTC: guanidinium thiocyanate
- GuHCl: guanidinium hydrochloride
- SPC: sodium perchlorate
- NaAc: sodium acetate
- CDV: canine distemper virus
- POX: vaccinia virus
- PBS: phosphate buffered saline
- Cq: quantification cycle
- 6-FAM: 6-carboxyfluorescin-phosphoramidit
- BHQ1: Black Hole Quencher 1

### Materials and Methods

The experiments to test the stabilizing effect of the different solutions were performed using cattle saliva as sample material, as this is a typical sample collected in a livestock. Saliva already contains intrinsic nucleases, which degrade nucleic acids. In order to increase the requirements for stabilization, Binuclease from GPROAN Protein Engineering (Cat.No. GPE0004) was added to the samples and incubation was performed at elevated temperatures (37 °C). As analytes, canine distemper viruses and vaccinia viruses (raised on vero cells and having titers of 10^{3.83} and 10^{3.5} per ml virus material, respectively) were added to the sample. Canine distemper virus contains single stranded RNA as genetic material, vaccinia virus uses double stranded DNA as genetic material. As the virus envelope in native viruses protects the genetic material from degradation through nucleases, the envelopes were destroyed through a heat treatment so that the genetic material was accessible to the nucleases. After incubation for 24 h at 37 °C, the viral RNA and DNA was extracted from the sample using the NucleoMag^{®} VET Kit from MACHEREY-NAGEL GmbH & Co.KG (Cat.No. 744200) on a KingFisher^{®} Duo Magnetic Particle Processor (Thermo Fisher Scientific). The RNA from the canine distemper virus was detected using an AgPath-ID^{™} One-Step RT-PCR Kit (Thermo Fisher Scientific Cat.No. 4387424) and specific primers and probe, the DNA from the vaccinia virus was detected using the QuantiTect Multiplex PCR Kit (Qiagen Cat.No. 204543) and specific primers and probe. Real time PCR and RT-PCR was performed on a C1000 Touch^{™}Thermal Cycler and CFX Real Time PCR detection system (BioRad).

The following NucleoMag^{®} VET buffers were used in the procedures:
Lysis buffer VL1
Binding buffer VEB
Wash buffer VEW1
Wash buffer VEW2
80% (v/v) aqueous ethanol (final washing step)
Elution buffer VEL
Proteinase K (>2,5 U/mg)

### Example 1: Stabilization of canine distemper viral RNA

Experimental setup:
- 20 µl of canine distemper virus (CDV) solution (raised on vero cells, titer 10^{3.83} per ml) in PBS was added to a 2 ml screw cap tube;
- the virus solution was heated to 95 °C for 5 min and then chilled on ice;
- 160 µl of stabilization solution was added;
- 60 µl cattle saliva and 10 µl (1000 U/ml) Binuclease were added; and
- samples were mixed and incubated for 24 h at 37 °C.

Reference: Instead of adding 160 µl stabilization solution, 160 µl lysis buffer VL1 from the NucleoMag^{®} Vet kit was added and samples were stored for 24 h at -20 °C. Samples were thawed immediately before extraction.

Extraction of RNA using NucleoMag^{®} VET Kit:
- 100 µl sample was added to a 96-well deep well block in row A;
- 20 µl proteinase K (>2,5 U/mg) and 100 µl buffer VL1 were added;
- incubation for 5 min at room temperature;
- 20 µl NucleoMag^{®} B-Beads and 350 µl buffer VEB were added;
- 600 µl wash buffer VEW1, VEW2 and 80% (v/v) ethanol were added to rows F, G and H of the 96-well block;
- 100 µl elution buffer VEL was added to the elution strip; and
- the 96-well block and the elution strip were put onto the appropriate position of a KingFischer Duo processor and the automated extraction protocol was started. Detection of CDV RNA: Analysis of the CDV RNA was performed on a C1000 Touch^{™}Thermal Cycler and CFX Real Time PCR detection system using the AgPath-ID^{™} One-Step RT-PCR Kit with the following CDV specific primers and probes:
   CDV forward primer - CTG TCR GTA ATC GAG RAT TCG A (SEQ ID NO:1)
   CDV reverse primer - GCC GAA AGA ATA TCC CCA GTT AG (SEQ ID NO:2)
   CDV probe - 6-FAM-ATC TTC GCC AGA RTC YTC AGT GCT-BHQ1 (SEQ ID NO:3). Amplification protocol: 10 min at 45 °C (reverse transcription); 10 min at 95 °C (activation of the Taq-polymerase); 45 cycles 15 s 95 °C (denaturation), 30 s 56 °C (annealing), 30 s 72 °C (elongation), fluorescence read after each cycle.

The following stabilization solutions were compared in example 1:

| Comparison 1: | Comparison 2: |
|---|---|
| Reference | Reference |
| 2.4 M GTC/GuHCl, 200 mM NaAc, pH6 | 2.4 M GTC/GuHCl, 200 mM NaAc, pH6 |
| 2.0 M GTC/GuHCl, 200 mM NaAc, pH6 | 2.0 M GTC/GuHCl, 200 mM NaAc, pH6 |
| 1.6 M GTC/GuHCl, 200 mM NaAc, pH6 | 1.6 M GTC/GuHCl, 200 mM NaAc, pH6 |
| 1.2 M GTC/GuHCl, 200 mM NaAc, pH6 | 1.2 M GTC/GuHCl, 200 mM NaAc, pH6 |
| 0.8 M GTC/GuHCl, 200 mM NaAc, pH6 | 0.8 M GTC/GuHCl, 200 mM NaAc; pH6 |
| 4.8 M GTC, 40 mM NaAc, pH6 | 4.8 M GuHCl, 40 mM NaAc, pH6 |
| 4.0 M GTC, 200 mM NaAc, pH6 | 4.0 M GuHCl, 200 mM NaAc, pH6 |
| 3.2 M GTC, 200 mM NaAc, pH6 | 3.2M GuHCl, 200 mM NaAc, pH6 |
| 2.4 M GTC, 200 mM NaAc, pH6 | 2.4 M GuHCl, 200 mM NaAc, pH6 |
| 2.0 M GTC, 200 mM NaAc, pH6 | 2.0 M GuHCl, 200 mM NaAc, pH6 |
| 1.6 M GTC, 200 mM NaAc, pH6 | 1.6 M GuHCl, 200 mM NaAc, pH6 |
| 1.2 M GTC, 200 mM NaAc, pH6 | 1.2 M GuHCl, 200 mM NaAc, pH6 |
| 0.8 M GTC, 200 mM NaAc, pH6 | 0.8 M GuHCl, 200 mM NaAc, pH6 |

In Comparison 1 the GTC/GuHCI mixtures are compared with GTC in Comparison 2 with GuHCl. Both Comparisons were performed two times using duplicate samples each time, therefore mean values of quadruplicate samples were evaluated. To evaluate the stabilization capabilities of the different solutions, the delta Cq-values of the individual solutions compared to the reference samples were calculated. Delta Cq-values below 1 were considered as insignificant changes. Only values >1 delta Cq compared to the reference were considered as significant degradation of the CDV RNA, as a delta Cq > 1 indicates, that more than 50% of the RNA was degraded during storage. The results are shown in Table 1.

**Table 1**

| Comparison 1 | Delta Cq/Reference | | Comparison 2 | Delta Cq/Reference |
|---|---|---|---|---|
| Reference | 0.00 | | Reference | 0.00 |
| 2.4 M GTC/GuHCI | 0.37 | | 2.4 M GTC/GuHCl | -0.18 |
| 2.0 M GTC/GuHCI | 0.27 | | 2.0 M GTC/GuHCl | 0.05 |
| 1.6 M GTC/GuHCI | 0.14 | | 1.6 M GTC/GuHCl | 0.43 |
| 1.2 M GTC/GuHCI | 0.68 | | 1.2 M GTC/GuHCl | 0.62 |
| 0.8 M GTC/GuHCI | 5.06 | | 0.8 M GTC/GuHCl | 7.58 |
| 4.8 M GTC | 0.15 | | 4.8 M GuHCl | 1.31 |
| 4.0 M GTC | 0.03 | | 4.0 M GuHCl | 3.23 |
| 3.2 M GTC | 0.44 | | 3.2 M GuHCl | 3.88 |
| 2.4 M GTC | 0.35 | | 2.4 M GuHCl | 3.89 |
| 2.0 M GTC | 1.09 | | 2.0 M GuHCl | 3.88 |
| 1.6 M GTC | 3.06 | | 1.6 M GuHCl | 3.24 |
| 1.2 M GTC | 4.91 | | 1.2 M GuHCl | 3.71 |
| 0.8 M GTC | 4.38 | | 0.8 M GuHCl | 8.13 |

Conclusion: In both comparisons, the equimolar solutions of guanidinium thiocyanate and guanidinium hydrochloride stabilized the RNA down to the concentration of 1.2 M. Only the lowest concentration of 0.8 M was not sufficient to stabilize the RNA. In contrast, each of the single salts did not show a stabilizing effect on the RNA for these low concentrations. A stabilizing effect for GTC can be seen with concentrations of 2.4 M and above. For GuHCl, even a 4.8 M solution was not capable to stabilize RNA in a sample. A combination of the two guanidinium salts at concentrations, where the single salts did not show any stabilizing effect can therefore lead to an effective stabilization solution.

Surprisingly it was found that the combination of the two salts has a synergistic effect on the stabilization property, so that even at concentrations, where the single salts show no effect, their combination leads to a sufficient stabilization effect. Due to this synergistic effect, it is possible to develop stabilization solutions based on combined guanidinium salts, which contain less hazardous material from each of the salts. It is therefore possible to develop a stabilization solution that is considered as nonhazardous due to its low content of the individual components.

### Example 2: Stabilization of vaccinia virus DNA

Experimental setup:
- 20 µl of vaccinia virus (POX) solution (raised on vero cells, titer 10^{3.5} per ml) in PBS was added to a 2 ml screw cap tube;
- the virus solution was heated to 95 °C for 5 min and then chilled on ice;
- 160 µl of stabilization solution was added;
- 60 µl cattle saliva and 10 µl (1000 U/ml) Binuclease were added; and
- samples were mixed and incubated for 48 h at 37 °C.

Reference: Instead of adding 160 µl stabilization solution, 160 µl lysis buffer VL1 from the NucleoMag^{®} Vet kit was added and samples were stored for 48 h at -20°C, samples were thawed immediately before extraction.

Extraction of DNA using NucleoMag^{®} VET Kit:
- 100 µl sample was added to a 96-well deep well block in row A;
- 20 µl proteinase K (> 2,5 U/mg) and 100 µl buffer VL1 were added;
- incubation for 5 min at room temperature;
- 20 µl NucleoMag^{®} B-Beads and 350 µl buffer VEB were added;
- 600 µl wash buffer VEW1, VEW2 and 80% (v/v) ethanol were added to rows F, G and H of the 96-well block;
- 100 µl elution buffer VEL was added to the elution strip; and
- the 96-well block and the elution strip were put onto the appropriate position of a KingFischer Duo processor and the automated extraction protocol was started

Detection of vaccinia virus DNA: Analysis of the DNA was performed on a C1000 Touch^{™}Thermal Cycler and CFX Real Time PCR detection system using the QuantiTect Multiplex PCR Kit with the following POX specific primers and probes:
POX forward primer - GCCAGAGATATCATAGCCGCTC (SEQ ID NO:4)
POX reverse primer - CAACGACTAACTAATTTGGAAAAAAAGAT (SEQ ID NO:5)
POX probe - 6-FAM-TTTTCCAACCTAAATAGAACTTCATCGTTGCGTT-BHQ1 (SEQ ID NO:6).

Amplification protocol: 15 min at 95 °C (activation of the Taq-polymerase); 45 cycles: 60 s 95 °C (denaturation), 30 s 60 °C (annealing), 30 s 72 °C (elongation), fluorescence read after each cycle.

The following stabilization solutions were investigated in experiment 2:
Reference
2.4 M GTC/GuHCl; 200 mM sodium acetate pH6
2.0 M GTC/GuHCl; 200 mM sodium acetate pH6
1.6 M GTC/GuHCl; 200 mM sodium acetate pH6
1.2 M GTC/GuHCl; 200 mM sodium acetate pH6

The results are shown in table 2.

**Table 2**

| | Delta Cq/Reference |
|---|---|
| Reference | 0.00 |
| 2.4 M GTC/GuHCl | -0.17 |
| 2.0 M GTC/GuHCl | -0.35 |
| 1.6 M GTC/GuHCl | -0.15 |
| 1.2 M GTC/GuHCl | -0.14 |

Conclusion: As shown for CDV RNA already, also the DNA from vaccinia virus gets stabilized by equimolar mixtures of guanidinium thiocyanate and guanidinium hydrochloride at concentrations between 1.2 and 2.4 M.

### Example 3: Stabilization of canine distemper viral RNA

In order to investigate the line between the concentration of equimolar solutions of guanidinium thiocyanate and guanidinium hydrochloride that stabilize CDV RNA (1.2 M) and the concentration that does not stabilize the RNA any more (0.8 M) an experiment was designed, which looks at this borderline at a higher resolution. Equimolar solutions of guanidinium thiocyanate and guanidinium hydrochloride with concentrations of 1.2 M, 1.0 M and 0.8 M were compared to the same concentrations of the single salts. Moreover, mixtures of guanidinium thiocyanate and guanidinium hydrochloride salt in unequal concentrations were investigated in order to prove, if only equimolar concentrations show a stabilizing effect or if the two salts may also be mixed in different concentrations.

Experimental setup:
- 20 µl of CDV solution (raised on vero cells, titer 10^{3.83} per ml) in PBS was added to a 2 ml screw cap tube;
- the virus solution was heated to 95 °C for 5 min and then chilled on ice;
- 160 µl of stabilization solution was added;
- 60 µl cattle saliva and 10 µl (1000 U/ml) Binuclease were added; and
- samples were mixed and incubated for 24 h at 37 °C.

Reference: Instead of adding 160 µl stabilization solution, 160 µl lysis buffer VL1 from the NucleoMag^{®} Vet kit was added and samples were stored for 24 h at -20 °C. Samples were thawed immediately before extraction. All samples were investigated in duplicates.

Extraction of RNA using NucleoMag^{®} VET Kit:
- 100 µl sample was added to a 96-well deep well block in row A;
- 20 µl proteinase K (> 2,5 U/mg) and 100 µl buffer VL1 were added;
- incubation for 5 min at room temperature;
- 20 µl NucleoMag^{®} B-Beads and 350 µl buffer VEB were added;
- 600 µl wash buffer VEW1, VEW2 and 80% (v/v) ethanol were added to rows F, G and H of the 96-well block;
- 100 µl elution buffer VEL was added to the elution strip; and
- the 96-well block and the elution strip were put onto the appropriate position of a KingFischer Duo processor and the automated extraction protocol was started.

Detection of CDV RNA: Analysis of the CDV RNA was performed on a C1000 Touch^{™}Thermal Cycler and CFX Real Time PCR detection system using the AgPath-ID^{™} One-Step RT-PCR Kit and the CDV specific primers and probes referred to in Example 1.

Amplification protocol: 10 min at 45 °C (reverse transcription); 10 min at 95 °C(activation of the Taq-polymerase); 45 cycles: 15 s 95 °C (denaturation), 30 s 56 °C (annealing), 30 s 72 °C (elongation), fluorescence read after each cycle The following stabilization solutions were compared in example 3:
Reference
1.2 M GTC, 200 mM NaAc, pH6
1.0 M GTC, 200 mM NaAc, pH6
0.8 M GTC, 200 mM NaAc, pH6
1.2 M GuHCl,200 mM NaAc, pH6
1.0 M GuHCl, 200 mM NaAc, pH6
0.8 M GuHCl, 200 mM NaAc, pH6
1.2 M GTC/GuHCl, 200 mM NaAc, pH6
1.0 M GTC/GuHCl, 200 mM NaAc, pH6
0.8 M GTC/GuHCl, 200 mM NaAc, pH6
2.4 M GTC/ 2.0 M GuHCl, 200 mM NaAc, pH6
2.0 M GTC/ 2.4 M GuHCl, 200 mM NaAc, pH6

Mean values of duplicate samples were evaluated. To evaluate the stabilization capabilities of the different solutions, the delta Cq-values of the individual solutions compared to the reference samples were calculated. Delta Cq-values below 1 were considered as insignificant changes. Only values >1 delta Cq compared to the reference were considered as significant degradation of the CDV RNA, as a delta Cq > 1 indicates, that more than 50% of the RNA was degraded during storage. The results are shown in Table 3.

**Table 3**

| Experiment 3 | Delta Cq/Reference |
|---|---|
| Reference | 0.00 |
| 1.2 M GTC | 3.08 |
| 1.0 M GTC | 2.84 |
| 0.8 M GTC | 2.74 |
| 1.2 M GuHCl | 2.30 |
| 1.0 M GuHCl | 1.91 |
| 0.8 M GuHCl | 2.52 |
| 1.2 M GTC/GuHCl | 0.41 |
| 1.0 M GTC/GuHCl | 3.39 |
| 0.8 M GTC/GuHCl | 3.41 |
| 2.4 M GTC/ 2.0 M GuHCl | -0.25 |
| 2.0 M GTC/ 2.4 M GuHCl | 0.02 |

Conclusion: Like shown before, the equimolar solution of guanidinium thiocyanate and guanidinium hydrochloride stabilized the RNA at a concentration of 1.2 M. Concentrations of 1.0 M or 0.8 M were not sufficient to stabilize the RNA. None of the single salts stabilized the RNA at the investigated concentrations. The unequal mixtures of 2.4 M GTC / 2.0 M GuHCl and 2.0 M GTC / 2.4 M GuHCl also stabilized the RNA, indicating that the two salts don't necessarily have to be mixed in equimolar concentrations.

### Example 4: Stabilization of canine distemper viral RNA using a mixture of three chaotrophic salts

The aim of the experiment was to investigate if a solution consisting of two chaotrophic salts with concentrations below the critical concentration for an efficient stabilization can be supplemented by a third chaotrophic salt in order to restore the stabilization properties.

Equimolar solutions of guanidinium thiocyanate and guanidinium hydrochloride with concentrations of 0,8 M were compared to solutions with the same concentrations of guanidinium salts plus sodium perchlorate (SPC) in concentrations of 0,8 M, 1,2 M, 1,6 M and 2,0 M respectively.

Experimental setup:
- 20 µl of canine distemper virus (CDV) solution (raised on vero cells, titer 10^{3.83} per ml) in PBS was added to a 2 ml screw cap tube;
- the virus solution was heated to 95 °C for 5 min and then chilled on ice;
   160 µl of stabilization solution was added;
   60 µl cattle saliva and 10 µl Binuclease (1000 U/ml) were added; and
   samples were mixed and incubated for 24 h at 37 °C.

Reference: Instead of adding 160 µl stabilization solution, 160 µl lysis buffer VL1 from the NucleoMag Vet kit was added and samples were stored for 24 h at -20 °C. Samples were thawed immediately before extraction. All samples were investigated in duplicates.

Extraction of RNA using NucleoMag^{®} VET Kit:
- 100 µl sample was added to a 96well deep well block in row A;
- 20 µl proteinase K (>2,5 U/mg) and 100 µl buffer VL1 were added;
- incubation for 5 min at room temperature;
- 20 µl NucleoMag^{®} B-Beads and 350 µl buffer VEB were added;
- 600 µl wash buffer VEW1, VEW2 and 80% (v/v) ethanol were added to rows F, G and H of the 96well block;
- 100 µl elution buffer VEL was added to the elution strip; and
- the 96-well block and the elution strip were put onto the appropriate position of a KingFischer Duo processor and the automated extraction protocol was started.

Detection of CDV RNA: Analysis of the CDV RNA was performed on a C1000 Touch^{™}Thermal Cycler and CFX Real Time PCR detection system using the AgPath-ID^{™} One-Step RT-PCR Kit with the CDV specific primers and probes referred to in Example 1.

Amplification protocol: 10 min at 45 °C (reverse transcription); 10 min at 95 °C (activation of the Taq-polymerase); 45 cycles: 15 s 95 °C (denaturation), 30 s 56 °C (annealing), 30 s 72 °C (elongation), fluorescence read after each cycle. The following stabilization solutions were compared in example 4:
Reference
0,8 M GTC/GuHCl; 200 mM sodium acetate pH6
0,8 M GTC/GuHCl/ 0,8 M SPC; 200 mM sodium acetate pH6
0,8 M GTC/GuHCl/ 1,2 M SPC; 200 mM sodium acetate pH6
0,8 M GTC/GuHCl/ 1,6 M SPC; 200 mM sodium acetate pH6
0,8 M GTC/GuHCl/ 2,0 M SPC; 200 mM sodium acetate pH6

Mean values of duplicate samples were evaluated. To evaluate the stabilization capabilities of the different solutions, the delta Cq-values of the individual solutions compared to the reference samples were calculated. Delta Cq-values below 1 were considered as insignificant changes. Only values >1 delta Cq compared to the reference were considered as significant degradation of the CDV RNA, as a delta Cq > 1 indicates, that more than 50% of the RNA was degraded during storage. The results are shown in Table 4.

**Table 4**

| Experiment 4 | Delta Cq/Reference |
|---|---|
| Reference | 0,00 |
| 0,8 M GTC/GuHCl | 2,89 |
| 0,8 M GTC/GuHCl/0,8 M SPC | 2,67 |
| 0,8 M GTC/GuHCl/1,2 M SPC | 2,54 |
| 0,8 M GTC/GuHCl/1,6 M SPC | 0,18 |
| 0,8 M GTC/GuHCl/2,0 M SPC | -0,29 |

## Claims

1. A method for stabilizing and isolating nucleic acids from a nucleic acid-containing biological sample which comprises adding to said sample, prior to subjecting it to a separation procedure, a sufficient amount of a stabilizing solution comprising a mixture of at least two chaotropic salts, wherein the sum of the molarity of said at least two chaotropic salts in the stabilizing solution is at least 2.2 M.

2. The method of claim 1 comprising
(a) providing the nucleic acid-containing sample,
(b) stabilizing the sample of step (a) by adding a sufficient amount of the stabilizing solution, optionally prior to, concomitant with, and/or after lysing the sample by the addition of a distinct lysis buffer and/or lysis solution;
(c) loading the stabilized sample of step (b) to a separation material to bind the nucleic acids to the separation material;
(d) washing the separation material with bound nucleic acids by adding one or more wash buffers to remove unwanted materials; and
(e) releasing the nucleic acids from the separation material by applying a release solution.

3. The method of claim 2, wherein the separation material is magnetic beads, step (d) comprises separating the beads from the sample by magnetic bead separation; and washing the separated beads with one or more wash buffers, and step (e) comprises releasing the nucleic acids from the separated beads by applying a release solution.

4. The method of any one of claims 1 to 3, wherein the stabilizing solution
(i) contains at least two chaotropic salts selected from barium, calcium, sodium and guanidinium perchlorates, thiocyanates, isothiocyanates, iodides and perchloracetates, and guanidinium hydrochlorid, preferably contains at least two guanidinium salts, most preferably contains a mixture of guanidinium thiocyanate and guanidinium hydrochloride; and/or
(ii) contains the alt least two chaotropic salts, at concentrations, selected independently from each other, from about 100 mM to about 5 M, preferably from about 500 mM to about 3 M, and most preferably from about 1.2 M to about 2.4 M; and/or
(iii) contains at least three chaotropic salts from those defined under (i) above, preferably contains a mixture of guanidinium thiocyanate, guanidinium hydrochloride and sodium perchlorate, with concentrations as defined under (ii) above, and/or
(iv) the sum of the molarity of said at least two chaotropic salts in the stabilizing solution is at least 2.2 M, preferably at least 2.4 M; and/or
(v) is added to the sample in an amount equal or greater than the volume of the sample, preferably about three-times the volume of the sample; and/or
(vi) further comprises buffers, salts, chelators, detergents, and/or reducing agents.

5. The method of claim 4, wherein the stabilizing solution contains
(i) guanidinium thiocyanate and guanidinium hydrochloride, independently from each other at concentrations from about 1.2 M to about 2.4 M; or
(ii) guanidinium thiocyanate, guanidinium hydrochloride and sodium perchlorate, independently from each other at concentrations from about 0.8 M to about 2.4 M.

6. The method of any one of claims 2 to 5, which comprises, prior to loading the stabilized sample to the separation material or beads of step (c), adding an inhibitor removal solution comprising (1) a polyvinylpyrrolidone (PVP) component containing PVP, a derivative thereof, and/or monomers thereof, and (2) ammonium sulfate (AS).

7. The method of claim 6, wherein the inhibitor removal solution
(i) contains the PVP component in an amount of about 2 to about 60 %(w/v), preferably in an amount of about 5 to about 30 %(w/v) and most preferably in an amount of about 8 to about 20 %(w/v); and/or
(ii) contains the AS in an amount of about 50 mM to about 5 M, preferably in an amount of about 100 mM to about 1.5 M, and most preferably in an amount of about 500 mM to about 1 M; and/or
(iii) weight ratio of PVP component to the AS is from about 10:1 to 1:10, preferably from 5:1 to about 1:5, and most preferably from about 2:1 to about 1:3; and/or
(iv) further comprises buffers, salts, chelators, detergents, reducing agents and/or stabilizing agents; and/or
(v) the volume of the inhibitor removal solution added to the sample in step (d) is equal or greater than the volume of the sample for separation, preferably about three-times the volume of the sample for separation.

8. The method of claim 6 or 7, wherein the PVP component comprises
(i) PVP with an average molecular weight of from about 1000 to about 500000 g/mol, preferably from about 2000 to about 50000 g/mol; and/or
(ii) alkylated or halogenated derivatives of polyvinylpyrrolidone.

9. The method of any one of claims 2 to 8, wherein
(i) the wash buffers of step (d) are salt-containing and/or ethanol-containing aqueous solutions; and/or
(ii) the release solution is water or an alkaline low-salt buffer

10. A stabilizing solution as defined in any one of claims 1 to 4.

11. A sample collection container comprising or being coated with the stabilizing solution as defined in any one of claims 1 to 4.

12. A kit comprising the stabilizing solution of claim 10 and/or the sample collection container of claim 11.

13. The kit of claim 12 further comprising one or more of lysis solutions/buffer, inhibitor removal solutions washing solutions, release solutions, including those defined in claims 6 to 9, protease-containing solutions, binding solutions and separation material/beads.

14. Use of the stabilizing solution of claim 10 or the sample collection container of claim 11 for stabilizing nucleic acids in biological samples.

15. Use of a kit of claim 12 or 13 for stabilizing nucleic acids in and isolating nucleic acids from biological samples.

## Amended claims

### Amended claims in accordance with Rule 137(2) EPC.

1. A method for stabilizing and isolating nucleic acids from a nucleic acid-containing biological sample which comprises adding to said sample, prior to subjecting it to a separation procedure, a sufficient amount of a stabilizing solution comprising a mixture of the two chaotropic salts guanidinium hydrochloride and guanidinium thiocyanate, wherein the sum of the molarity of said two chaotropic salts in the stabilizing solution is at least 2.4 M, and wherein the stabilizing solution contains each of said chaotropic salts, independently from each other, at concentrations from 1.2 M to 2.4 M.

2. The method of claim 1 comprising
(a) providing the nucleic acid-containing sample,
(b) stabilizing the sample of step (a) by adding a sufficient amount of the stabilizing solution, optionally prior to, concomitant with, and/or after lysing the sample by the addition of a distinct lysis buffer and/or lysis solution;
(c) loading the stabilized sample of step (b) to a separation material to bind the nucleic acids to the separation material;
(d) washing the separation material with bound nucleic acids by adding one or more wash buffers to remove unwanted materials; and
(e) releasing the nucleic acids from the separation material by applying a release solution.

3. The method of claim 2, wherein the separation material is magnetic beads, step (d) comprises separating the beads from the sample by magnetic bead separation; and washing the separated beads with one or more wash buffers, and step (e) comprises releasing the nucleic acids from the separated beads by applying a release solution.

4. The method of any one of claims 1 to 3, wherein the stabilizing solution further contains chaotropic salts selected from barium, calcium, and sodium perchlorates, thiocyanates, isothiocyanates, iodides and perchloracetates, and guanidinium perchlorate, isothiocyanate, iodide and perchloracetate.

5. The method of any one of claims 1 to 4, wherein the stabilizing solution contains at least a third chaotropic salts from those defined in claim 4, preferably contains a mixture of guanidinium thiocyanate, guanidinium hydrochloride and sodium perchlorate.

6. The method of claim 4 or 5, wherein the stabilizing solution further contains sodium perchlorate, at a concentration from 0.8 M to 2.4 M.

7. The method of any one of claims 1 to 6, wherein the stabilizing solution is added to the sample in an amount equal or greater than the volume of the sample, preferably about three-times the volume of the sample.

8. The method of any one of claims 1 to 7, wherein the stabilizing solution further comprises buffers, salts, chelators, detergents, and/or reducing agents.

9. The method of any one of claims 2 to 8, wherein
(i) the wash buffers of step (d) are salt-containing and/or ethanol-containing aqueous solutions; and/or
(ii) the release solution is water or an alkaline low-salt buffer

10. A stabilizing solution as defined in any one of claims 1 to 4.

11. A sample collection container comprising or being coated with the stabilizing solution as defined in any one of claims 1 to 4.

12. A kit comprising the stabilizing solution of claim 10 and/or the sample collection container of claim 11.

13. The kit of claim 12 further comprising one or more of lysis solutions/buffer, washing solutions, release solutions, including those defined in claim 9, protease-containing solutions, binding solutions and separation material/beads.

14. Use of the stabilizing solution of claim 10 or the sample collection container of claim 11 for stabilizing nucleic acids in biological samples.

15. Use of a kit of claim 12 or 13 for stabilizing nucleic acids in and isolating nucleic acids from biological samples.
